(19) Europäisches Patentamt / European Patent Office / Office européen des brevets

(11) **EP 4 712 040 A1**

(12) **EUROPEAN PATENT APPLICATION**

(43) Date of publication:
**18.03.2026 Bulletin 2026/12**

(21) Application number: 25202308.0

(22) Date of filing: **15.09.2025**

(51) International Patent Classification (IPC):
**G06V 10/25** (2022.01)    **G01N 33/44** (2006.01)
**G06V 10/44** (2022.01)    **G06V 20/52** (2022.01)
**G06V 20/60** (2022.01)    **C14B 17/00** (2006.01)

(52) Cooperative Patent Classification (CPC):
**G06V 20/60; C14B 17/005; G01N 33/447;
G06V 10/25; G06V 10/457; G06V 20/52**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH LA MA MD TN**

(30) Priority: **13.09.2024 IT 202400020506**

(71) Applicant: **WEGA S.R.L.
36071 Arzignano (VI) (IT)**

(72) Inventor: **FILIPPOZZI, Riccardo
36070 Brogliano (VI) (IT)**

(74) Representative: **Caldon, Giuliano et al
Gallo & Partners S.r.l.
Via Rezzonico, 6
35131 Padova (IT)**

(54) **METHOD FOR DETERMINING ONE OR MORE PARAMETERS RELATING TO ONE OR MORE HIDES AND SYSTEMS IMPLEMENTING THIS METHOD**

(57)    The present invention refers to a method for determining one or more parameters of one or more leathers (L1-L5). The method comprises the steps of: - generating a surfaces vector S; (a) acquiring a representation constituted by a first vector R0 of N positions of values related to a first strip (F1) affected by the presence of said one or more leathers (L1-L5), each value of the first vector R0 representing a value of presence or absence of said one or more leathers (L1-L5) detected at N areas aligned within the first strip (F1); (b) acquiring a representation constituted by a second vector R1 of N positions of values related to a second strip (F2) affected by the presence of said one or more leathers (L1-L5), each value of the second vector R1 representing a value of presence or absence of said one or more leathers (L1-L5) detected at N areas aligned within the second strip (F2); - in a first processing block, checking the continuity of said one or more leathers (L1-L5) by comparing the values of the two vectors R0, R1 and in case of continuity assigning a corresponding common leather identifier within the second vector R1 and updating the surfaces vector S; - in a second processing block assigning an identifier to the positions of the second vector R1 with presence value but lacking common identifier; - in a third processing block, creating an identifiers vector of said one or more leathers (L1-L5), determining an end-of-leather event, providing the values of said one or more parameters and updating the surfaces vector S again.

FIG. 7

**Description**

TECHNICAL FIELD OF THE INVENTION

The present invention relates to a method for determining one or more parameters related to one or more leathers in systems for processing and/or treating and/or checking leathers.

More particularly, the present invention relates to a method for determining the number of leathers and/or the area of said one or more leathers in systems for processing and/or treating and/or checking leathers. The invention also relates to a system which implements such method.

DESCRIPTION OF THE STATE OF THE ART

Known in the field of production of leathers is the need to move the same through different stations which carry out, on the leathers themselves, different types of processing and/or treatments and/or quality controls.

The production of the leathers, whether they are natural or synthetic, require various steps that necessarily involve the movement of the same along different stations dedicated to ad hoc mechanical or chemical processing or treatments within a suitable plant.

Only as a non-limiting example, the splitting machines are mentioned which are used in the tanning industry for separating the precious external part (full-grain leather) of an animal hide from the less precious part (split leather), or finishing plants that - starting from lowquality leathers - apply to the same one or more finished layers of synthetic material, possibly colored.

In addition to the treatments which intervene mechanically and/or chemically on the leather, suitable stations are then provided for within the plants, adapted for checking the correct operation of the process, for example by detecting suitable parameters within the leather. For example, evaluation units can be provided for evaluating the surface quality of the leather, generally by means of the use of cameras which are directed on the surface of the leather and/or which detect possible deformities/defects.

Known within such plants is the use of control systems substantially constituted by optical detectors or image detectors, for example cameras or digital scanners, capable of detecting the surface characteristics of the leather/leathers at predetermined areas of detection/scanning of the leather/leathers. Such detectors are based on the acquisition of a digitized representation of images and their digital representation is suitably processed, for example through suitable algorithms, in order to identify parameters for checking surface quality, such as the presence of defects, undesired color variations, etc.

In said plants, the various leathers to be treated are initially manually loaded by an operator who places them spread out, one alongside the other, in a feed station of the plant that is typically constituted by a movable surface (conveyor belt).

A parameter of considerable importance in the treatments of leathers relates to the spatial geometric identification of the leathers that cross the plant. The spatial geometric identification can be configured in the simple determination/count of the number of treated leathers, or number of leathers to be treated, or in the determination of the area of the leather/leathers.

According to the prior art, such operation is assigned to the operator who identifies/counts the leathers within the plant or to empirical evaluation systems for evaluating their areas.

The main object of the present invention is therefore that of proposing a solution that allows obtaining the determination of one or more parameters related to the spatial geometric identification of the leather/leathers that feed and/or cross a plant for treating such leather/leathers.

DESCRIPTION OF THE PRESENT INVENTION

In a first aspect thereof, the present invention refers to a method for determining one or more parameters of one or more leathers, in which said method comprises the steps of:

- generating a surfaces vector S;

    (a) acquiring a representation constituted by a first vector R0 of N positions of values related to a first strip affected by the presence of said one or more leathers, each value of said first vector R0 representing a value of presence or absence of said one or more leathers detected at N areas aligned within said first strip;
    (b) acquiring a representation constituted by a second vector R1 of N positions of values related to a second strip affected by the presence of said one or more leathers, each value of said second vector R1 representing a value of presence or absence of said one or more leathers detected at N areas aligned within said second strip;

- in a first processing block checking the continuity of said one or more leathers by comparing the values of said two vectors R0, R1 and in case of continuity assigning a corresponding common leather identifier within said second vector R1 and updating said surfaces vector S;
- in a second processing block assigning an identifier to the positions of said second vector R1 with presence value but lacking common identifier;
- in a third processing block creating an identifiers vector of said one or more leathers, determining an end-of-leather event, providing the values of said one or more parameters and updating said surfaces vector S again.

Preferably, said one or more parameters comprise the number of leathers and/or the area of said one or more leathers, the area of said one or more leathers being obtained from the surfaces vector S.

In a preferred embodiment, said first block comprises the following steps:

(a) setting the following variables NL=0, LP=1, SID=2;
(b) acquiring a new vector NR;
(c) updating the vectors R0, R1: R0=R1 and R1=NR;
(d) setting an index IP=0;
(e) as long as IP<N then:

(e1) fixing L=R0[IP] and C=R1[IP];
(e2) if L!=C and L>= SID and C>= LP then:

(e21) if C>=SID then:

(e211) overwriting each position in R0 and R1 which has value L with the value C;
(e212) fixing S[C-SID]+=S[L-SID]
and fixing S[L-SID]=0;
(e213) assigning, to all the elements of R1 that are in contact on the right and left with R1 [IP], an identifier value equal to L as long as the values of the elements of R1[IP] are greater than or equal to LP;
(e214) going to step (e3);

(e22) fixing C=L and R1[IP]=L and going to step (e213);

(e3) increasing IP by one unit and returning to step (e).

According to a preferred embodiment, said second block comprises the following steps:

(a) setting an index IP=0;
(b) as long as IP<N then:

(b1) if R1[IP]==LP then:

(b11) assigning, to C, a new identifier Id;
(b12) fixing R1[IP]=C;
(b13) assigning, to all the elements of R1 that are in contact on the right and left with R1[IP], said new identifier C as long as the values of the elements of R1[IP] are greater than
or equal to LP and going to step (b2);

(b2) increasing IP by one unit and returning to step (b).

Preferably, said third block comprises the following steps:

(a) creating a vector IDLIST;
(b) setting an index IP=0;
(c) as long as IP<N then:

(c1) fixing CID=R0[IP];
(c2) if CID>=SID and CID is not present in IDLIST then:

    (c21) if CID is not present in R1 then:

        (c211) signaling end-of-leather event;
        (c212) providing the value S[CID-SID];
        (c213) fixing S[CID-SID]=0;

    (c22) saving CID in the vector IDLIST;

    (c3) increasing IP by one unit and returning to step (c);

(d) setting an index IP=0;
(e) as long as IP<N then:

    (e1) if R1[IP]>=SID then:
    (e11) fixing S[R1[IP]-SID]+=1;
    (e2) increasing IP by one unit and returning to step (e);

(f) returning to step (b) of the first block.

Preferably, the step (e213) of the first block or the step (b13) of the second block comprises the following steps:

(a) fixing I=IP;
(b) as long as I>0 and R1[I] != NL then:

    (b1) fixing P=R1[I-1];
    (b2) if P!=C and P>=LP then:
    (b21) fixing R1[I-1]=C;
    (b3) decreasing I by one unit and returning to step (b);

(c) fixing I=IP;
(d) as long as I<(N-1) and (R1[I] != NL) then:

    (d1) fixing P=R1[I+1];
    (d2) if P!=C and P>=LP then:
    (d21) fixing R1[I+1]=C;
    (b3) increasing I by one unit and returning to step (d);

(e) end.

In a preferred embodiment, said first strip and said second strip are extended along respective longitudinal directions, said first strip and said second strip being parallel to each other and immediately adjacent to each other. In particular, the first strip and the second strip correspond to respective strips of the leather that are immediately adjacent (and parallel to each other).

According to a preferred embodiment, said longitudinal directions of the first strip and of the second strip run perpendicular to a movement direction of said one or more leathers.

Preferably, said first strip and said second strip coincide (on a fixed abutment surface) and the detection of the N areas within the first strip and the detection of the N areas within the second strip occur in successive instants of time while said one or more leathers are advanced along a movement direction.

In a preferred embodiment, the detection of the N areas within the first strip and/or the detection of the N areas within the second strip occurs with said one or more leathers placed on a surface.

According to a first preferred embodiment, said surface is a slidable surface and the detection of the N areas within the first strip and/or the detection of the N areas within the second strip occurs by means of optical detection means adapted to affect a corresponding strip of said one or more leathers placed on said surface.

Preferably, the optical detection means comprise a line-scan camera.

In a second preferred embodiment, said surface is stopped and the detection of the N areas within the first strip and/or the detection of the N areas within the second strip occurs by means of optical detection means adapted to affect the first and the second strip of said one or more leathers placed on said surface.

According to a preferred embodiment, the optical detection means comprise a camera.

Preferably, the method comprises a step of displaying said one or more parameters determined starting from said surfaces vector S on an appropriate user interface.

In another aspect thereof, the present invention refers to a system for determining one or more parameters of one or more leathers, said system implementing the above-described method, in which said system comprises:

- optical detection means adapted to inspect at least one strip which affects said one or more leathers;
- a processing unit adapted to process the values detected by said optical detection means in order to determine said one or more parameters;
- a user interface adapted to display said one or more parameters determined by said processing unit.

BRIEF DESCRIPTION OF THE FIGURES

Further advantages, objectives and characteristics as well as embodiments of the present invention are defined in the claims and will be clarified hereinbelow by means of the following description, in which reference is made to the enclosed drawing tables; in the drawings, component parts and/or characteristics corresponding or equivalent of the present invention are identified by the same reference numbers. In particular:

- figure 1 represents a schematic view of a detection unit of the leathers according to a preferred embodiment of the invention;
- figures 2 to 5 show different operating steps of the unit of figure 1 during its operation;
- figure 6 shows an embodiment variant of the unit of figure 1;
- figure 7 shows, in schematic form, an acquisition of the leathers by means of the unit according to the present invention;
- figure 8 represents the block diagram of the processing according to the method of the present invention;
- figures 9 to 11 represent, in detail, several blocks of figure 8;
- figure 12 represents, in detail, a block of figure 9 and figure 10.

DETAILED DESCRIPTION OF PREFERRED EMBODIMENTS OF THE PRESENT INVENTION

Even if the present invention is described hereinbelow with reference to its embodiments represented in the drawing tables, the present invention is not limited to the embodiments described hereinbelow and represented in the drawing tables.

On the contrary, the embodiments described and represented in the drawing tables clarify several aspects of the present invention, the object of which being defined by the claims.

The present invention has particular but not exclusive application in the tanning field for the treatment of leather of natural origin, or hides.

In particular, the method according to the present invention can be advantageously used within a plant, for example at the start, end or as intermediate unit between successive stations dedicated for the processing of the leather, e.g. mechanical or chemical treatment stations.

It should in any case be specified that the use of the movement device is not limited to such applications. On the contrary, the present invention has convenient application in any type of situation where the determination of geometric parameters of the leather/leathers is requested, such as their count or the determination of their area.

In addition, in the following description and with reference to the figures, the parts constituting the plant related to the treatment processes per se will not be described, rather focus will be centered on the single zone of the plant in which the application of the method according to the invention is implemented.

For the sake of description simplicity, in the following present description the parts of the plant adapted to allow the implementation of the method according to the invention will be indicated in their entirety with the reference letter A and therefore shown and considered substantially as a separate unit A preferably usable within a plant for treating leathers.

The objective of the method according to the invention is that of automatically identifying the characteristic parameters of the leather/leathers with reference in particular to their geometric form, more particularly the number of leathers and/or the area of the leather/leathers. It is known that in the treatment plants, the leathers are loaded into a feed zone and are preferably placed spread out and manually separated from each other by an operator.

Then leathers then cross the various stations of the plant and typically maintain their mutual position.

In the figures, by way of example, a possible configuration is shown which the leathers can take on: five leathers L1-L5 are identified, placed spread out and separated from each other above an abutment surface 100.

The abutment surface 100 is preferably of slidable type 100 (conveyor belt 100) and allows the movement of the leathers L1-L5 along an advancement direction D1.

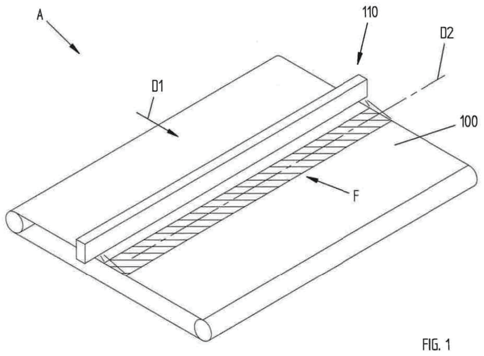

For the correct operation of the method of the invention capable of identifying the leathers, it is essential that the leathers do not touch each other and/or are not superimposed on each other for any section. In a preferred embodiment, the unit A according to the invention is provided with optical detection means 110. The detection means 110 are configured for inspecting an underlying strip F, as indicated in figure 1.

Such strip F is extended longitudinally preferably along a direction D2 perpendicular to the advancement direction D1 of the leathers L1-L5. For example, in a preferred embodiment, the detection means 100 comprise a single camera or a camera placed at a suitable distance with respect to the abutment surface 100 and are capable of identifying an inspection strip F that affects the abutment surface 100 for its entire width.

For example, the first detection means 110 comprise a line-scan camera or, more preferably, a plurality of aligned cameras for covering the scanning of the underlying area substantially for the entire width of the abutment surface 100.

In another preferred embodiment, the detection means can comprise a plurality of devices, one next to the other, each adapted for identifying an inspection area that is narrow with respect to the width of the abutment surface 100.

In a further embodiment, the first detection means can comprise a device adapted to identify an inspection area that is narrow with respect to the width of the abutment surface and then movement means of the device itself in order to allow the sliding of the detection means in the direction of the width of the abutment surface.

The function of the unit A according to the invention is that of acquiring/detecting, in succession, two parallel strips F1, F2 affected by the leathers L1-L5.

In the illustrated embodiment, the two strips F1, F2 are longitudinally extended in respective directions perpendicular to the advancement direction D1 of the leathers L1-L5.

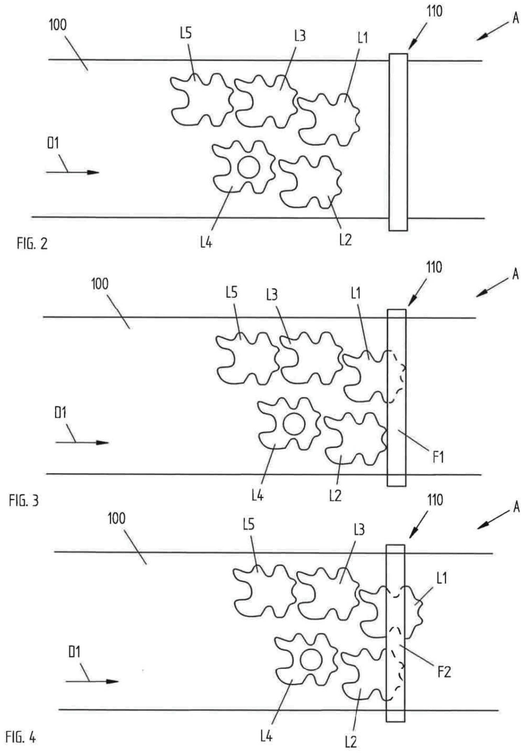

The first strip F1 is acquired in a first instant of time t1 by the detection means 110, for example as shown in figure 3, and the second strip F2 is acquired in a successive instant of time t2 by the detection means 110 after the leathers L1-L5 are made to advance by the conveyor belt 100 by a section, for example as shown in figure 4.

It is evident that in an embodiment variant, it can be the detection means to be move above the leathers L1-L5, preferably maintained stopped, for example by stopping the underlying abutment surface.

The first strip F1 and the second strip F2 are extended along respective longitudinal directions, the first strip F1 and the second strip F2 being parallel to each other and immediately adjacent to each other. Preferably, the longitudinal directions of the first strip F1 and of the second strip F2 run perpendicular to the advancement direction D1 of the leathers L1-L5.

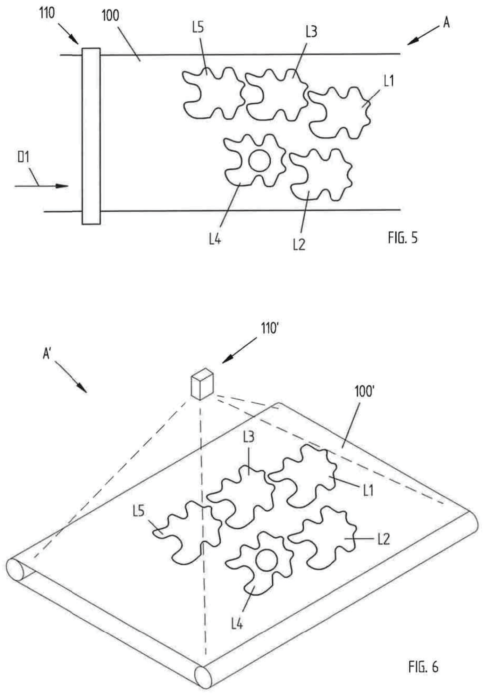

In a further embodiment variant, the two strips F1, F2 can be identified starting from a single image of the spread-out leathers L1-L5 and by means of a post-processing of said image. A preferred embodiment of a unit A' which allows such identification of the two strips F1, F2 is shown by way of example in figure 6 in which the optical detection means 110' comprise a camera capable of acquiring an image of all the leathers placed on the underlying abutment surface 100'. The detection of the image by means of the optical detection means 110' will preferably occur in an instant of time in which the abutment surface 100' is maintained at least temporarily stopped.

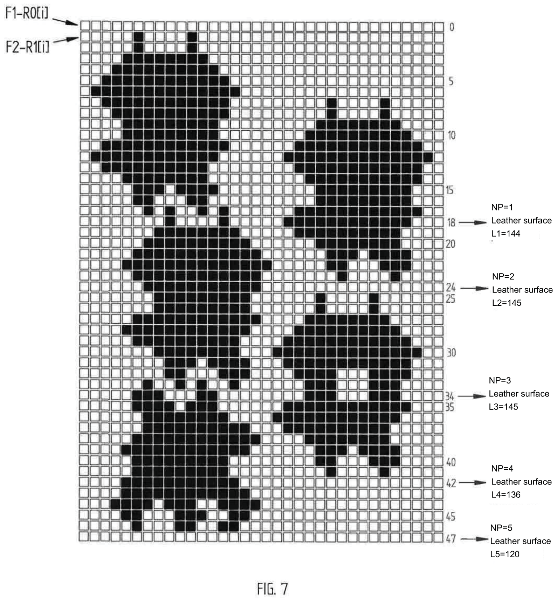

Within a strip F, F1, F2, the detection means 110 are capable of detecting the presence or absence of a leather at N positions aligned within the strip F, F1, F2 itself. By way of example, the detection means 100 of the invention shown in the figure are capable of detecting the presence of leather at 34 positions aligned within the strip F, F1, F2 (better visible in figure 7). Such 34 positions are identified by respective areas and the detection means 110 are capable of identifying if there is the leather within each area. The higher the number of positions/areas used, the greater the precision in the successive processing operations. In preferred embodiments, the number of positions is selected within a range comprised between 64 and 136.

In the illustrated embodiment, the areas are preferably constituted by small squares. The embodiment variants, the areas could have different geometric shape. The detection means are preferably capable of identifying and providing the size value Wi of such detected areas, for example in terms of square centimeters of each area. Preferably such areas are all equal to each other. The size value Wi of such areas is subsequently used for determining the characteristic parameter of the leathers L1-L5 constituted by the area of each leather.

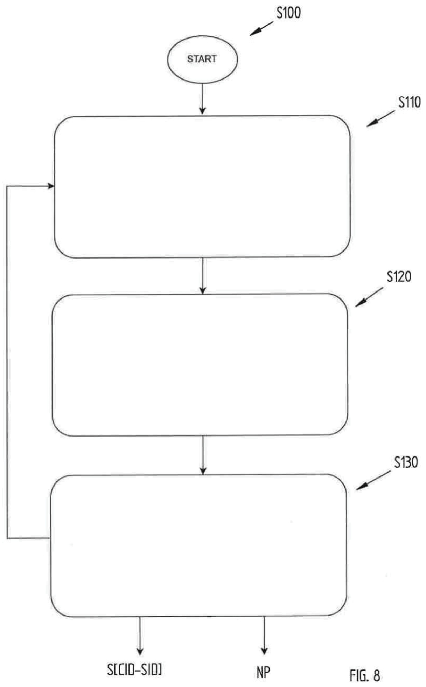

The detected values of the two strips F1, F2 are associated, by a suitable processing unit, a respective vectors R0 and R1 and such values are used, according to one aspect of the present invention, in a processing method adapted to determine the parameters of interest related to the leathers L1-L5, i.e. the number and/or the area of the leather/leathers. In addition, according to the present invention, the two strips F1, F2 are acquired in succession and the detected values are used for overwriting and updating, each time, the two vectors R0 and R1 with the new acquisition up to the end of the acquisition of the leathers L1-L5, the latter situation being indicated for example in figure 5 where all the leathers L1-L5 have been inspected by the detection means 110. Figure 7 schematically shows the acquisition with strips of several leathers through the detection means 100, 100'. Each detected strip is subsequently associated with the two vectors of R0 and R1 which are subjected to processing according to the method described below with reference to the flow diagrams pursuant to figures 8 to 12.

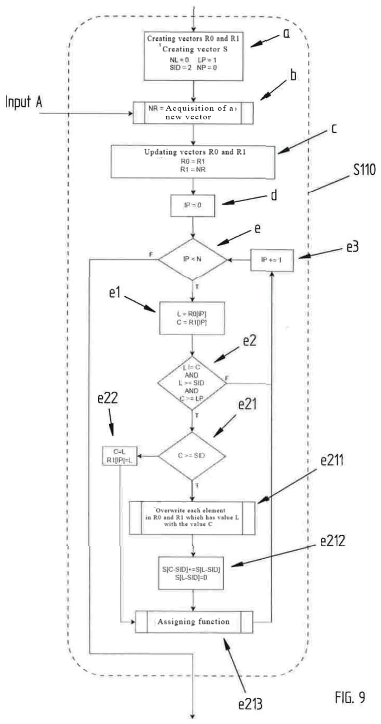

The flow diagram of figure 8 indicates the general block scheme of the processing according to the present invention and each block is described in detail in the flow diagrams of figure 9, 10 and 11.

The processing starts in step S100.

With regard to that stated above, a representation is acquired, constituted by a first vector R0 of N positions of values related to a first strip F1 affected by the presence of one or more leathers L1-L5, in which each value of the first vector R0 represents a value of presence or absence of said one or more leathers L1-L5 detected at N areas aligned within the first strip F1 and a representation is acquired that is constituted by a second vector R1 of N positions of values related to a second strip F2 affected by the presence of said one or more leathers L1-L5, in which each value of the second vector R1 represents a value of presence or absence of said one or more leathers L1-L5 detected at N areas aligned within the second strip F2.

The presence value within a vector R0, R1 is conventionally selected equal to 1 while the absence value is conventionally selected equal to 0. In embodiment variants, other values can be selected.

In a first processing block S110, a surfaces vector S is generated in which the values contained therein are associated with one of said parameters of interest related to the leathers L1-L5, in particular with the area of the leather/leathers.

In such first processing block S110 the continuity of said one or more leathers L1-L5 is checked by comparing the values of the two vectors R0, R1 and in case of continuity a corresponding identifier ID of common leather is assigned within the second vector R1. The identifier ID is conventionally selected with values greater than or equal to 2 such to not be confused with the values of presence or absence within the two vectors R0, R1. In embodiment variants, other values can be selected.

In such first processing block S110, also said surfaces vector S is updated.

By way of example, the effect of the first block S110 sui two vectors R0, R1 è the following:

before the execution of the first block S110

$$R0 = [0\ 0\ 0\ 0\ 2\ 2\ 2\ 0\ 0\ 0\ 0\ 0]$$

$$R1 = [0\ 0\ 1\ 1\ 1\ 1\ 1\ 1\ 0\ 1\ 1\ 0]$$

after the execution of the first block S110

$$R0 = [0\ 0\ 0\ 0\ 2\ 2\ 2\ 0\ 0\ 0\ 0\ 0]$$

$$R1 - [0\ 0\ 2\ 2\ 2\ 2\ 2\ 2\ 0\ 1\ 1\ 0]$$

In a second processing block S120, an identifier is assigned to the positions of the second vector R1 with presence value but lacking common identifier.

By way of example, the effect of the second block S110 on the two vectors R0, R1 is the following:

before the execution of the first block S110

$$R0 = [0\ 0\ 0\ 0\ 2\ 2\ 2\ 0\ 0\ 0\ 0\ 0]$$

$$R1 = [0\ 0\ 2\ 2\ 2\ 2\ 2\ 2\ 0\ 1\ 1\ 0]$$

after the execution of the first block S110

$$R0 = [0\ 0\ 0\ 0\ 2\ 2\ 2\ 0\ 0\ 0\ 0\ 0]$$

$$R1 = [0\ 0\ 2\ 2\ 2\ 2\ 2\ 2\ 0\ 3\ 3\ 0]$$

In a third processing block S130, an identifiers vector IDLIST of said one or more leathers L1-L5 is created. In particular, the identifiers vector IDLIST contains the aforesaid identifiers, more particularly both the identifiers ID of common leather within the second vector R1, and the identifiers of the positions of the second vector R1 with presence value but lacking common identifier.

The identifiers vector IDLIST is used determining the end-of-leather event. The end-of-leather event indicates that that no second strip F2 is actually affected by the leather and therefore the leather itself has been completely inspected.

The end-of-leather event preferably allows increasing the counter of leathers NP, indicating the number of inspected leathers.

Following the end event, in addition, a parameter is preferably provided at the output indicated with S[CID-SID], from which it will be possible to calculate the area of the just-inspected leather, as better described hereinbelow.

Advantageously, the parameter S[i] is the value of the component of the surfaces vector S associated with the aforesaid just-inspected leather. More particularly, the parameter S[i] represents the value of the component of the surfaces vector S having index "i".

In particular, the parameter S[CID-SID] represents the final value of parameter S[i] which is obtained following the end-of-leather event. Within the third block S130, then, the values of the surfaces vector S are processed and updated.

In particular, the updating of the surfaces vector S is carried out by updating the value of the parameter S[i].

More particularly, the value of the parameter S[i] is updated, if the relative conditions are respected, with each iteration of the first and second block, specifically up to reach the final value represented by S[CID-SID].

By way of example, in figure 7, the values are indicated which progressively taken on NP and S[CID-SID] during the detection in succession of the strips on the leathers L1-L5.

The first block S110, according to that illustrated in figure 9, comprises the following steps:

(a) setting the following variables NL=0, LP=1, SID=2, NP=0;
(b) acquiring a new vector NR;
(c) updating the vectors R0, R1: R0=R1 and R1=NR;
(d) setting an index IP=0;
(e) as long as IP<N then:

(e1) fixing L=R0[IP] and C=R1[IP];
(e2) if L!=C and L>= SID and C>= LP then:

(e21) if C>=SID then:

(e211) overwriting each position in R0 and R1 which has value L with the value C;
(e212) fixing S[C-SID]+=S[L-SID]
and fixing S[L-SID]=0;
(e213) assigning, to all the elements of R1 that are in contact on the right and left with R1 [IP], an identifier value equal to L as long as the values of the elements of R1[IP] are greater than or equal to LP;
(e214) going to step (e3);

(e22) fixing C=L and R1[IP]=L and going to step (e213);

(e3) increasing IP by one unit and returning to step (e). By way of example, the effect of the step (e213) of assigning, to all the elements of R1 that are in contact on the right and left with R1[IP], an identifier value equal to L is the following:

before the execution of the step (e213)

$$R1 = [0\ 0\ 1\ 1\ 2\ 1\ 1\ 1\ 0\ 1\ 1\ 0]$$

after the execution of the step (e213)

$$R1 = [0\ 0\ 2\ 2\ 2\ 2\ 2\ 2\ 0\ 1\ 1\ 0]$$

or in a second example:

before the execution of the step (e213)

$$R1 = [0\ 0\ 2\ 2\ 2\ 2\ 2\ 2\ 0\ 3\ 1\ 0]$$

after the execution of the step (e213)

$$R1 - [0\ 0\ 2\ 2\ 2\ 2\ 2\ 2\ 0\ 3\ 3\ 0].$$

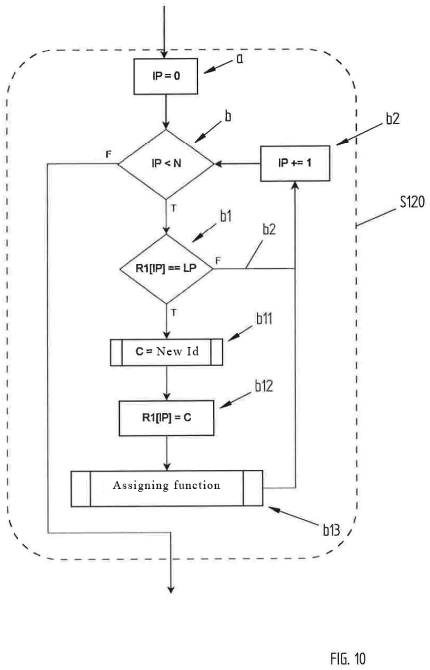

The second block S120, according to that illustrated in figure 10, comprises the following steps:

(a) setting an index IP=0;
(b) as long as IP<N then:

(b1) if R1[IP]==LP then:

(b11) assigning a C a new identifier Id;
(b12) fixing R1[IP]=C;
(b13) assigning, to all the elements of R1 that are in contact on the right and left with R1[IP], said new identifier C as long as the values of the elements of R1[IP] are greater than or equal to LP and going to step (b2);

(b2) increasing IP by one unit and returning to step (b).

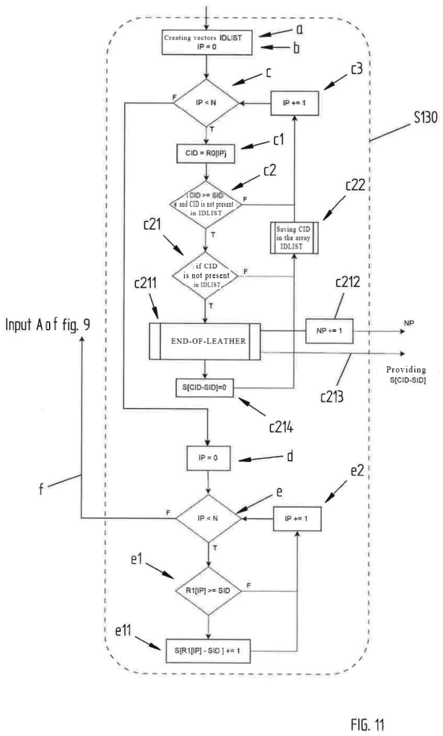

The third block S130, according to that illustrated in figure 11, comprises the following steps:

(a) creating a vector IDLIST;
(b) setting an index IP=0;
(c) as long as IP<N then:

(c1) fixing CID=R0[IP];
(c2) if CID>=SID and CID is not present in IDLIST then:

(c21) if CID is not present in R1 then:

(c211) signaling end-of-leather event;
(c212) increasing NP by one unit and providing the value of NP;
(c213) providing the value S[CID-SID];
(c214) fixing S[CID-SID]=0;

(c22) saving CID in the vector IDLIST;

(c3) increasing IP by one unit and returning to step (c);

(d) setting an index IP=0;
(e) as long as IP<N then:

(e1) if R1[IP]>=SID then:
(e11) fixing S[R1[IP]-SID]+=1;
(e2) increasing IP by one unit and returning to step (e);

(f) returning to step (b) of the first block S110.

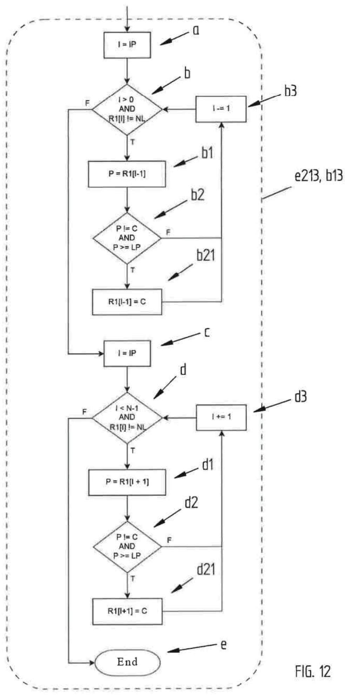

The step (e213) of the first block S110 and the step (b13) of the second block S120, also indicated as "Assigning function" in the respective figures of page 9 and 10, is illustrated in figure 12 and preferably comprises the following steps:

(a) fixing I=IP;
(b) as long as I>0 and R1[I] != NL then:

(b1) fixing P=R1[I-1];
(b2) if P!=C and P>=LP then:
(b21) fixing R1[I-1]=C;
(b3) decreasing I by one unit and returning to step (b);

(c) fixing I=IP;
(d) as long as I<(N-1) and (R1[I] != NL) then:

(d1) fixing P=R1[I+1];
(d2) if P!=C and P>=LP then:
(d21) fixing R1[I+1]=C;
(b3) increasing I by one unit and returning to step (d);

(e) end.

At the output of the third block S130, as stated above, the parameter NP is provided which indicates the value of inspected and identified leathers.

At the output of the third block S130, in addition, the parameter S[CID-SID] is provided that is related to the just-inspected leather. The value identified by the parameter S[CID-SID] corresponding to the number of total areas detected and counted for the just-inspected leather. In other words, the parameter S[CID-SID] indicates the number of total areas detected (total small squares) constituting the just-inspected leather. The area of such leather will then be simply obtained by multiplying the value of such parameter by the size value Wi of each area.

For example, there will be an area equal to:

$$S[CID\text{-}SID]*Wi = 20*10dm^2 = 200dm^2$$

having presumed a numerical value equal to 20 for S[CID-SID] and $10dm^2$ for Wi.

Due to the above-described method, therefore, at the end of the detection method, it is possible to identify the number of detected leathers L1-L5 and/or the area of each leather L1-L5.

The number of detected leathers L1-L5 and/or the area of each leather L1-L5 are preferably displayed in an appropriate user interface, not shown, constituted for example by a display or a monitor.

Advantageously, due to the above-described method, it is possible to identify the correct surface area of a leather also in case of a hole in the same, such as for example the leather L4 in the figure.

By means of the preceding detailed description, it is therefore demonstrated that the method according to the present invention allows reaching the pre-established objects. In particular, the method according to the present invention allows determining the parameters of one or more leathers within a plant for treating such leathers.

Even if the present invention has been clarified above by means of the detailed description of its embodiments, represented in the drawing tables, the present invention is not limited to the embodiments represented in the drawing tables and described above.

On the contrary, the object of the present invention is defined by the claims.

## Claims

1. Method for determining one or more parameters of one or more leathers (L1-L5); wherein said one or more parameters comprise at least the area of said one or more leathers (L1-L5); said method comprising:

- generating a surfaces vector (S), wherein the values contained in said surfaces vector (S) are associated with the areas of said leathers (L1-L5); said area of said one or more leathers (L1-L5) being obtained from said surfaces vector (S);

(a) acquiring a representation consisting of a first vector R0 of N positions of values relating to a first strip (F1) affected by the presence of said one or more leathers (L1-L5), each value of said first vector R0 representing a value of presence or absence of said one or more leathers (L1-L5) detected at N areas aligned within said first strip (F1);
(b) acquiring a representation consisting of a second vector R1 of N positions of values relating to a second strip (F2) affected by the presence of said one or more leathers (L1-L5), each value of said second vector R1 representing a value of presence or absence of said one or more leathers (L1-L5) detected at N areas aligned within said second strip (F2); wherein said first strip (F1) and said second strip (F2) are extended along respective longitudinal directions, said first strip (F1) and said second strip (F2) being parallel to each other and immediately adjacent to each other;

- in a first processing block, checking the continuity of said one or more leathers (L1-L5) by comparing the values of said two vectors R0, R1 and, in case of continuity, assigning a corresponding common leather identifier within said second vector R1 and updating said surface vector S;
- in a second processing block, assigning an identifier to the positions of said second vector R1 with a presence value but without a common identifier;
- in a third processing block,

creating a vector of identifiers (IDLIST) containing said identifiers of said one or more leathers (L1-L5), determining an end-of-leather event, using said identifiers vector (IDLIST); wherein, following said end-of-leather event, a parameter (S[CID-SID]) is provided at the output, from which the area of said just-inspected leather is calculated; wherein the identified value of said parameter (S[CID-SID]) corresponding to the number of total areas detected and counted for said just-inspected leather; wherein the area of said leather is obtained by multiplying the value of said parameter (S[CID-SID]) by a size value (Wi),

providing at least said the area of said one or more leathers (L1-L5) of values of said one or more parameters and updating said surface vector S again.

2. Method according to claim 1, **characterized in that** said first block comprises the following steps:

(a) setting the following variables NL=0, LP=1, SID=2, NP=0;
(b) acquiring a new vector NR;
(c) updating vectors R0, R1: R0=R1 and R1=NR;
(d) setting an index IP=0;
(e) as long as IP<N then:

(e1) fixing L=R0[IP] and C=R1[IP];
(e2) if L!=C and L>= SID and C>= LP then:
(e21) if C>=SID then:

(e211) overwriting each position in R0 and R1 that has value L with value C;
(e212) fixing S[C-SID]+=S[L-SID]
and fixing S[L-SID]=0;
(e213) assigning to all the elements of R1 that are in contact on the right and left with R1[IP] an identifier value equal to L as long as the values of the elements of R1[IP] are greater than or equal to LP;
(e214) going to step (e3);
(e22) fixing C=L and R1[IP]=L and going to step (e213);
(e3) increasing IP by one unit and returning to step (e).

3. Method according to claim 2, **characterized in that** said second block comprises the following steps:

(a) setting an index IP=0;
(b) as long as IP<N then:

(b1) if R1[IP]==LP then:
(b11) assigning to C a new identifier Id;
(b12) fixing R1[IP]=C;
(b13) assigning to all the elements of R1 that are in contact on the right and left with R1[IP] said new identifier C as long as the values of the elements of R1[IP] are greater than or equal to LP and going to step (b2);
(b2) increasing IP by one unit and returning to step (b).

4. Method according to claim 3, **characterized in that** said third block comprises the following steps:

(a) creating a vector IDLIST;
(b) setting an index IP=0;
(c) as long as IP<N then:

(c1) fixing CID=R0[IP];
(c2) if CID>=SID and CID is not present in IDLIST then:

**EP 4 712 040 A1**

(c21) if CID is not present in R1 then:
(c211) signaling end-of-leather event;
(c212) increasing NP by one unit and providing the value of NP;
(c213) providing the value S[CID-SID];
(c214) fixing S[CID-SID]=0;
(c22) saving CID in the vector IDLIST;
(c3) increasing IP by one unit and returning to step (c);

(d) setting an index IP=0;
(e) as long as IP<N then:
(e1) if R1[IP]>=SID then:

(e11) fixing S[R1[IP]-SID]+=1;
(e2) increasing IP by one unit and returning to step (e);

(f) returning to step (b) of claim 2.

5. Method according to claim 2, **characterized in that** said step (e213) comprises the following steps:

(a) fixing I=IP;
(b) as long as I>0 and R1[I] != NL then:

(b1) fixing P=R1[I-1];
(b2) if P!=C and P>=LP then:

(b21) fixing R1[I-1]=C;
(b3) decreasing I by one unit and returning to step (b);

(c) fixing I=IP;
(d) as long as I<(N-1) and (R1[I] != NL) then:

(d1) fixing P=R1[I+1];
(d2) if P!=C and P>=LP then:
(d21) fixing R1[I+1]=C;
(b3) increasing I by one unit and returning to step (d);

(e) end.

6. Method according to claim 3, **characterized in that** said step (b13) comprises the following steps:

(a) fixing I=IP;
(b) as long as I>0 and R1[I] != NL then:

(b1) fixing P=R1[I-1];
(b2) if P!=C and P>=LP then:
(b21) fixing R1[I-1]=C;
(b3) decreasing I by one unit and returning to step (b);

(c) fixing I=IP;
(d) as long as I<(N-1) and (R1[I] != NL) then:

(d1) fixing P=R1[I+1];
(d2) if P!=C and P>=LP then:
(d21) fixing R1[I+1]=C;
(b3) increasing I by one unit and returning to step (d);

(e) end.

7. Method according to any one of the preceding claims, **characterized in that** said longitudinal directions of said first strip (F1) and said second strip (F2) run perpendicular to a movement direction (D1) of said one or more leathers (L1-L5).

8. Method according to any one of claims 1 to 6, **characterized in that** the detection of said N areas within said first strip (F1) and the detection of said N areas within said second strip (F2) occur in successive instants of time while said one or more leathers (L1-L5) are advanced along a movement direction (D1).

9. Method according to any one of the preceding claims, **characterized in that** the detection of said N areas within said first strip (F1) and/or the detection of said N areas within said second strip (F2) occurs with said one or more leathers (L1-L5) placed in a surface (100; 100').

10. Method according to claim 9, **characterized in that** said surface (100) is a slidable surface and said detection of said N areas within said first strip (F1) and/or the detection of said N areas within said second strip (F2) occurs by means of optical detection means (110; 110') adapted to affect a corresponding strip of said one or more leathers (L1-L5) arranged on said surface.

11. Method according to claim 10, **characterized in that** said optical detection means (110') comprise a line-scan camera.

12. Method according to claim 9, **characterized in that** said surface (100') is stationary and said detection of said N areas within said first strip (F1) and/or the detection of said N areas within said second strip (F2) occurs by means of optical detection means (110') adapted to affect said first and said second strips (F2) of said one or more leathers (L1-L5) placed on said surface.

13. Method according to claim 12, **characterized in that** said optical detection means (110') comprise a camera.

14. Method according to any one of the preceding claims, **characterized in that** it comprises a step of displaying said one or more parameters determined starting from said surface vector S on an appropriate user interface.

15. System for determining one or more parameters of one or more leathers (L1-L5), said system implementing the method according to any one of the preceding claims; **characterized in that** it comprises:

- optical detection means (110; 110') adapted to inspect at least one strip affected by said one or more leathers (L1-L5);
- a processing unit adapted to process the values detected by said optical detection means (110; 110') to determine said one or more parameters;
- a user interface adapted to display said one or more parameters determined by said processing unit.

FIG. 1

100    L5        L3      L1    110    A

D1

FIG. 2    L4        L2

100    L5    L3    L1    110    A

D1    L4    L2    F1

FIG. 3

100    L5        L3    110    A

L1

D1    F2

FIG. 4    L4    L2

FIG. 5

FIG. 6

FIG. 7

FIG. 8

FIG. 9

FIG. 10

FIG. 11

FIG. 12

Europäisches Patentamt
European Patent Office
Office européen des brevets

# EUROPEAN SEARCH REPORT

**Application Number**

EP 25 20 2308

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2023/333026 A1 (MASTROTTO ROBERTO [IT] ET AL) 19 October 2023 (2023-10-19) * paragraphs [0080] - [0164]; figures 2, 5, 10, 12, 13 * | 1-15 | INV. G06V10/25 G01N33/44 G06V10/44 G06V20/52 |
| A | WO 2021/240279 A1 (CONCERIA PASUBIO S P A [IT]) 2 December 2021 (2021-12-02) * page 4, line 26 - page 7, line 27; figure 7a * | 1-15 | G06V20/60 C14B17/00 |
| A | WO 2011/030360 A1 (CAMOGA SPA [IT]; MASCETTI MATTEO [IT]) 17 March 2011 (2011-03-17) * page 12 - page 18; figure 2 * | 1-15 | |

**TECHNICAL FIELDS SEARCHED (IPC)**

G06V
G01N
C14B

The present search report has been drawn up for all claims

| Place of search | Date of completion of the search | Examiner |
|---|---|---|
| The Hague | 16 January 2026 | Craciun, Paula |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 25 20 2308

This annex lists the patent family members relating to the patent documents cited in the above-mentioned European search report.
The members are as contained in the European Patent Office EDP file on
The European Patent Office is in no way liable for these particulars which are merely given for the purpose of information.

16-01-2026

| Patent document cited in search report | | Publication date | Patent family member(s) | | Publication date |
|---|---|---|---|---|---|
| US 2023333026 | A1 | 19-10-2023 | CA | 3195055 A1 | 17-03-2022 |
| | | | EP | 4211275 A1 | 19-07-2023 |
| | | | US | 2023333026 A1 | 19-10-2023 |
| | | | WO | 2022053906 A1 | 17-03-2022 |
| WO 2021240279 | A1 | 02-12-2021 | EP | 4158590 A1 | 05-04-2023 |
| | | | WO | 2021240279 A1 | 02-12-2021 |
| WO 2011030360 | A1 | 17-03-2011 | TW | 201115138 A | 01-05-2011 |
| | | | WO | 2011030360 A1 | 17-03-2011 |

EPO FORM P0459

For more details about this annex : see Official Journal of the European Patent Office, No. 12/82